# EUROPEAN PATENT APPLICATION

(11) **EP 1 982 724 A1**
(43) Date of publication of application: **22.10.2008**
(21) Application number: 06843635.1
(22) Date of filing: 28.12.2006
(51) Int. Cl.: A61K 36/899, A23L 1/30, A23L 1/308, A61K 31/716, A61P 3/04, A61P 3/06, A61P 3/10, A61P 9/12, C08B 37/00

(54) **THERAPEUTIC AGENT FOR METABOLIC SYNDROME AND FOOD CONTAINING THE THERAPEUTIC AGENT**

(30) Priority: 06.01.2006 JP 2006001677; 06.10.2006 JP 2006275502
(71) Applicant: Sapporo Breweries Limited, Tokyo 150-8522 (JP)
(72) Inventor: KIHARA, Makoto, 37-1, Nitta Kizaki-machi Ota-shiGunma 370-0393 (JP); ARAKI, Shigeki, 10, Okatohme Yaizu-shi Shizuoka 425-0013 (JP); SHIMIZU, Chikako, 10, Okatohme Yaizu-shi Shizuoka 425-0013 (JP); NAKAMURA, Yoshiyuki, Ota-shi Gunma 370-0393 (JP); ITO, Kazutoshi, 37-1, Nitta Kizaki-machi Ota-shi Gunma 370-0393 (JP); IKEGAMI, Sachie, Chiyoda-ku Tokyo 102-8357 (JP); AOE, Seiichiro, Chiyoda-ku Tokyo 102-8357 (JP)
(74) Representative: Vossius & Partner
(86) International application number: PCT/JP2006/326257
(87) International publication number: WO 2007/077929

(57) **Abstract**

It is an object of the invention to provide a novel therapeutic agent that can reduce visceral fat accumulation to prevent or treat metabolic syndrome. In order to achieve this object, the invention provides a therapeutic agent for metabolic syndrome that comprises, as an active ingredient, dietary fiber aggregation in which water-soluble β-glucan has the highest content. Particularly effective as the water-soluble β-glucan is water-soluble β-glucan having a β-1,3 glycoside bond and β-1,4 glycoside bond in the same molecule.

## Description

### Technical Field

The present invention relates to a therapeutic agent for metabolic syndrome and to foods containing it.

### Background Art

Obesity is a condition characterized by excess accumulation of body fat, usually resulting from lifestyle habits such as excessive intake of nutrients and lack of exercise. With the drastic increase in the number of obese individuals, and in light of the fact that the condition of obesity leads to such problems as diabetes, 1 hypertension and hyperlipidemia, its treatment has become an issue of national interest in Japan.

In recent years, the metabolic syndrome (pathological condition that tends to cause arteriosclerosis) has been defined in Japan as well (Non-patent document 1), and waist circumference has been adopted as one of its essential criteria. Obesity is treated primarily by diet and exercise, with drug therapy also being employed in cases where such measures are not easily applicable for some reason.

[Non-patent document 1] Journal of the Japanese Society of Internal Medicine, 94(4), 794-809, 2005

### Disclosure or the Invention

### Problem to be Solved by the Invention

The drugs currently in use for drug therapy (therapeutic agents for obesity such as anorectic agents or metabolism-increasing drugs) are problematic due to concerns about their side-effects.

It is therefore an object of the present invention to provide a novel therapeutic agent that can reduce visceral fat accumulation to prevent or treat metabolic syndrome, as well as foods containing the agent.

### Means for Solving the Problem

In order to achieve the object stated above, the invention provides a therapeutic agent for metabolic syndrome that comprises, as an active ingredient, dietary fiber aggregation in which water-soluble β-glucan has the highest content.

The therapeutic agent for metabolic syndrome according to the invention is characterized in that dietary fiber aggregation (polysaccharide aggregates) is an active ingredient thereof, and that water-soluble β-glucan has the highest content in the dietary fiber aggregation. Such a constitution can effectively inhibit elevation of cholesterol (especially non-HDL cholesterol, which is known as "bad cholesterol") in the serum and liver and of triglycerides in the liver, thus providing a notable inhibiting effect on visceral fat accumulation.

In other words, the therapeutic agent for metabolic syndrome of the invention can be used as a therapeutic agent with a reducing effect on visceral fat (as a visceral fat reducer) and/or as a therapeutic agent with an inhibiting effect on visceral fat accumulation (as a visceral fat accumulation inhibitor), and can additionally be used as a therapeutic agent with an inhibiting effect on adipocyte hypertrophy (as an adipocyte hypertrophy inhibitor).

The water-soluble β-glucan is preferably water-soluble β-glucan having a β-1,3 glycoside bond and β-1,4 glycoside bond in the same molecule (a glucan with a β-1,3 glycoside bond will hereinafter also be referred to as a "β-1,3-glucan", and this will be applied correspondingly for glucans with glycoside bonds at other positions). The dietary fiber aggregation is preferably obtained from one or more sources selected from the group consisting of barley, oats, rye and adlay Cell walls of plant cells, algae (e.g. laminaria), yeast, bacteria, mold and mushroom, for example, are abundant in β-glucan, and using water-soluble β-glucan derived from one or more of these groups will allow the content of water-soluble β-glucan with a β-1,3 glycoside bond and β-1,4 glycoside bond in the same molecule to be easily increased.

If the water-soluble β-glucan content is 20% to 100% by weight relative to the total weight of the dietary fiber aggregation, the therapeutic agent is particularly effective as a therapeutic agent for metabolic syndrome. That is, while water-soluble β-glucan alone can be the active ingredient of the therapeutic agent for metabolic syndrome, the effect of reducing cholesterol and visceral fat and other effects become more pronounced if the content is at least 20% by weight. As other dietary fibers in dietary fiber aggregation with a β-glucan content of less than 100% by weight, there may be mentioned β-1,4-glucan (cellulose), hemicellulose, lignin, pectin and the like.

The therapeutic agent for metabolic syndrome may be added to foods, and foods containing the therapeutic agent for metabolic syndrome exhibit the effect of reducing cholesterol and visceral fat and the effect of inhibiting accumulation thereof.

### Effects of the Invention

The therapeutic agent for metabolic syndrome of the invention and foods containing it can reduce visceral fat accumulation to prevent or treat metabolic syndrome.

### Brief Description of the Drawings

Fig. 1 is a graph showing changes in total serum cholesterol with time in placebo group and test group.
Fig. 2 is a graph showing changes in serum LDL cholesterol with time in placebo group and test group.
Fig. 3 is a graph showing changes in body weight with time in placebo group and test group.
Fig. 4 is a graph showing changes in BMI with time in placebo group and test group.
Fig. 5 is a graph showing changes in waist circumference with time in placebo group and test group.
Fig. 6 is a graph showing changes in visceral fat with time in placebo group and test group.
Fig. 7 is a graph showing changes in subcutaneous fat with time in placebo group and test group.
Fig. 8 is a graph showing changes in total body fat (visceral fat + subcutaneous fat) with time in placebo group and test group.

### Best Modes for Carrying Out the Invention

Preferred embodiments of the invention will now be described in detail.

As used herein, the term "metabolic syndrome" refers to the condition defined by "Definition and Criteria of Metabolic Syndrome" (Journal of the Japanese Society of Internal Medicine, 94(4), 794-809, 2005) by the Examination Committee for Criteria for Metabolic Syndrome.

Specifically, for Japanese, the basic diagnostic index is a waist circumference of ≥ 85 cm for males and ≥ 90 cm for females (corresponding to a visceral fat area of ≥ 100 cm² for both males and females). In addition to this waist circumference index, the diagnostic criteria include the following:
(1) abnormal lipoprotein levels: high triglycerides (triglyceride level of ≥ 150 mg/dL) and/or low HDL cholesterol (HDL cholesterol level of < 40 mg/dL);
(2) high blood pressure: systolic pressure of ≥ 130 mmHg and/or diastolic pressure of ≥ 85 mmHg;
(3) high blood sugar (fasting glucose level of ≥ 110 mg/dL).

As used herein, the term "dietary fiber" means "any indigestible food component that cannot be digested by human digestive enzymes" (Standard Tables of Food Composition in Japan, 5th Revised Edition). The "dietary fiber aggregation" includes one or more types of such dietary fiber.

The dietary fiber aggregation may include, in addition to water-soluble β-glucan as the essential component of the therapeutic agent for metabolic syndrome of the invention, also β-1,4-glucan (cellulose), hemicellulose, pectin, lignin, insulin, chitin (chitosan), guar gum, glucomannan, galactomannan, seaweed polysaccharides (alginic acid, laminarin, fucoidin, carrageenan), arabinoxylan, arabinogalactan and the like.

The water-soluble β-glucan is most preferably water-soluble β-glucan having a β-1,3 glycoside bond and β-1,4 glycoside bond in the same molecule (also referred to hereinafter as "(1→3),(1→4)-β-D-glucan"), but as examples thereof, in addition to this water-soluble β-glucan, there may also be mentioned water-soluble β-1,3-glucan with a branch at the 6-position (water-soluble β-glucan having a β-1,3 glycoside bond and β-1,6 glycoside bond in the same molecule). The content of water-soluble β-1,3-glucan with a branch at the 6-position is preferably less than the content of (1→3),(1→4)-β-D-glucan, and it is also preferred that only (1→3),(1→4)-β-D-glucan is contained as the water-soluble β-glucan.

As examples of the water-soluble β-1,3-glucan with a branch at the 6-position, there may be mentioned β-glucans found in mushrooms such as shiitake mushroom, oyster mushroom, nameko mushroom, winter mushroom, matsutake mushroom, hon-shimeji mushroom, split gill, Crepidotus mollis, elm mushroom, maitake mushroom, turkey tail, tinder polypore, straw mushroom, Jew's ear, lingzhi, bracket fungus, long net stinkhorn, cauliflower mushroom, agaricus mushroom and the like.

The dietary fiber aggregation is preferably obtained from cereal such as barley, oats, rye or adlay. Barley and oats are preferred among these, and most preferred is waxy barley, which tend to have a high water-soluble β-glucan content.

The content of water-soluble β-glucan (especially (1→3),(1→4)-β-D-glucan) is preferably 20% to 100% by weight, more preferably 30% to 100% by weight, even more preferably 40% to 100% by weight and most preferably 50% to 100% by weight, relative to the total weight of the dietary fiber aggregation. Examples of quantification methods for individual β-glucan are provided by AOAC International (AOAC Official Method 995.16).

The method for obtaining the dietary fiber aggregation may be any publicly known method, and for example, seeds are preferably used to obtain (1→3),(1→4)-β-D-glucan from barley or the like.

A test for obesity can be performed by feeding a high-cholesterol diet to rats for a prescribed period of time and then measuring the cholesterol levels and organ weights. As a result of the test using the therapeutic agent for metabolic syndrome of the invention, the therapeutic agent for metabolic syndrome of the invention exhibited the effect of inhibiting elevation of non-HDL cholesterol as well as the effect of increasing HDL cholesterol and preventing accumulation of visceral fat (reducing visceral fat and/or inhibiting visceral fat accumulation). The therapeutic agent for metabolic syndrome of the invention is therefore particularly effective for treatment of metabolic syndrome.

The therapeutic agent for metabolic syndrome may be used as an additive to food. As examples of foods to which it may be added, there may be mentioned grains such as rice and barley rice, and flours such as wheat flour. Specifically, such foods include noodles, bread and confectioneries made from wheat flour. They also include processed foods made from wheat, barley or the like (for example, miso and shoyu). The amount of the therapeutic agent for metabolic syndrome in a food may be any amount that allows the therapeutic agent to function as an active ingredient, but it is preferably at least 5% by weight, more preferably at least 10% by weight, even more preferably at least 20% by weight and most preferably at least 30% by weight, relative to the total weight of the food.

Since barley and the like also contain β-glucan in the inner layer (endosperm) of the seed, the whole grains may be used directly as a food, but alternatively the outer layer of the seed may be polished for use as a food or for addition to a food (or food material). The seeds may be milled into flour or used as grains without milling. They are preferably added as powder to foods (noodles, bread, confectioneries, etc.) for processing, or mixed as grains with rice for boiling. Also, (1→3),(1→4)-β-D-glucan is water-soluble dietary fiber and can be easily extracted, and it can therefore be used in the form of an extract.

### Examples

The present invention will now be explained in greater detail based on examples and comparative examples below, with the understanding that these examples are in no way limitative on the invention.

### (Example 1: Cholesterol elevation-inhibiting effect of barley in high cholesterol diet-fed rats)

Four-week-old Fischer 344 male rats (Charles River Laboratories Japan, Inc.) were used as the experimental animals. After 5 days of preliminary feeding with a purified diet containing no cholesterol or sodium cholate, the rats were divided into 4 groups of 8 each, and the test diet was fed for 2 weeks.

The compositions of the diets used for the feeding were based on the composition (modified AIN-93G) shown in Table 1 below, and 0.5% cholesterol and 0.15% sodium cholate were added to create a high-cholesterol diet. As for the lipid source, 10% lard was contained instead of 7% soybean oil (see "Project Report on Rat Response to High-Cholesterol Diet", Journal of the Japanese Society of Nutrition and Food Science, 45, 6, 564-567, 1992).

**[Table 1]**

| Diet (modified AIN-93G) | Weight |
|---|---|
| Casein | 200 |
| L-Cystine | 3 |
| α-Cornstarch | 499.486 |
| Sucrose | 100 |
| Lard | 100 |
| Cellulose | 50 |
| AIN-93G mineral mixture | 35 |
| AIN-93 vitamin mixture | 10 |
| Choline bitartrate | 2.5 |
| t-Butylhydroquinone | 0.014 |
| Total | 1000 |

Polished rice, wheat (unpolished) and two types of barley (unpolished) to be added to the diets were boiled. They were then freeze-dried, and ground with a cyclone mill into polished rice flour, wheat flour and barley flour, respectively, for addition to the diets. With regard to polished rice group as the control group, all of the α-cornstarch was replaced with polished rice-derived carbohydrate, and the polished rice-derived protein, lipid and total dietary fiber contents were adjusted. With regard to wheat group and barley group, the diet was formulated to contain 5% total dietary fiber, the protein, lipid and total dietary fiber contents were adjusted, and the total weight was adjusted to be 1000 g using α-cornstarch.

The results of the formulation were as follows: The (1→3),(1→4)-β-D-glucan contents in the samples were 0.16% for polished rice group, 0.19% for wheat group, 2.41% for barley group 1 and 2.59% for barley group 2. That is, the proportions of (1→3),(1→4)-β-D-glucan in the dietary fiber of the samples were: polished rice group = 3.2%, wheat group = 3.8%, barley group 1 = 48.2%, barley group 2 = 51.8%. Only with regard to barley group 1 and barley group 2, (1→3),(1→4)-β-D-glucan has the highest content in the dietary fiber.

The rats were reared in an environment with a temperature of 23 ± 2°C, humidity of 55 ± 5% and light/dark cycle of 12 hours, and the diet and water were made freely available.

The results are shown in Tables 2, 3, 4 and 5. Table 2 shows initial body weight (g), final body weight (g), body weight gain (g/d), feed intake (g/d) and feed efficiency (%) for the rats in each group. The initial body weight (g) is the weight of each rat at the start of feeding, the final body weight (g) is the weight of each rat at the end of 2 weeks of feeding, the body weight gain (g/d) is the body weight increase per day of each rat during the 2-week feeding period, the feed intake (g/d) is the dietary intake per day of each rat during the 2-week feeding period, and the feed efficiency (%) is the ratio of feed intake to body weight gain.

**[Table 2]**

| | Polished rice | Wheat | Barley 1 | Barley 2 |
|---|---|---|---|---|
| Initial body weight (g) | 81.0 ± 1.9 | 81.0 ± 2.8 | 81.1 ± 2.4 | 81.0 ± 2-0 |
| Final body weight (g) | 144.6 ± 5.8 | 147.5 ± 6.2 | 144.9 ± 5.8 | 145.0 ± 4.7 |
| Body weight gain (g/d) | 4.5 ± 0.3 | 4.8 ± 0.3 | 4.6 ± 0.3 | 4.6 ± 0.2 |
| Feed intake (g/d) | 10.8 ± 0.4 | 11.0 ± 0.4 | 10.7 ± 0.4 | 10.7 ± 0.6 |
| Feed efficiency (%) | 42.2 ± 1.6 | 43.2 ± 1.9 | 42.7 ± 1.5 | 42.8 ± 2.1 |

| | | | | |
|---|---|---|---|---|
| There are no significant differences between the groups for any of the items. The numerical values represent mean ± standard deviation. | | | | |

According to the results, no difference was found between the groups in terms of body weight gain and other items during the 2-week feeding period (Table 2).

Table 3 shows the values of serochemical parameters of the rats in each group.

**[Table 3]**

| | Polished rice * | Wheat | Barley 1 | Barley 2 |
|---|---|---|---|---|
| Total cholesterol (mg/dl) | 198.8 ± 38.7^{a} | 162.7 ± 27.8^{ab} | 149.9 ± 28.8^{b} | 137.2 ± 28.7^{b} |
| HDL cholesterol (mg/dl) | 16.5 ± 1.1^{a} | 25.3 ± 1.7^{b} | 29.3 ± 5.0^{b} | 27.1 ± 4.3^{b} |
| Non-HDL cholesterol (mg/dl) | 182.3 ± 38.88^{a} | 137.4 ± 28.9^{ab} | 120.6 ± 29.4^{b} | 110.1 ± 32.1^{b} |
| Triglyceride (mg/dl) | 40.3 ± 7.1 | 39.5 ± 7.1 | 45.6 ± 10.4 | 42.6 ± 8.5 |

| | | | | |
|---|---|---|---|---|
| There are significant differences (p < 0.05) between the numerical values with different alphabet superscripts. The numerical values represent mean ± standard deviation. * For polished rice group, n = 7 due to poor blood sampling; for the other groups, n=8. | | | | |

In the table, there are significant differences (p < 0.05) between the numerical values with alphabet superscripts. No statistically significant difference is found between the numerical values with no alphabet superscripts.

According to the results, total serum cholesterol levels were significantly low in barley groups compared to polished rice group, and in particular, the levels of non-HDL cholesterol, which is known as bad cholesterol were significantly low in barley groups (Table 3). On the other hand, barley groups had the highest HDL cholesterol levels.

Table 4 shows hepatic lipid levels of the rats in each group.

**[Table 4]**

| | Polished rice | Wheat | Barley 1 | Barley 2 |
|---|---|---|---|---|
| Total cholesterol, | | | | |
| (mg/ liver) | 272.2 ± 28.3^{a} | 257.4 ± 18.5^{a} | 201.2 ± 16.6^{b} | 191.3 ± 12.7^{b} |
| (mg/g liver) | 48.9 ± 2_{.}7^{a} | 44.0 ± 1.7^{b} | 35.2 ± 1.6^{c} | 34.8 ± 2.4^{c} |
| Triglycerides | | | | |
| (mg/ liver) | 181.3 ± 18.5^{a} | 182.2 ± 21.5^{a} | 137.5 ± 22.4^{b} | 131.1 ± 21.8^{b} |
| (mg/g liver) | 32.6 ± 2.1^{a} | 31.2 ± 3.6^{a} | 24.0 ± 3.4^{b} | 23.8 ± 3.6^{b} |
| Phospholipids | | | | |
| (mg/ liver) | 141.4 ± 20.2 | 142.5 ± 12.6 | 135.9 ± 13.7 | 126.1 ±10.5 |
| (mg/g liver) | 25.4 ± 2.5^{a} | 24.3 ± 1.8^{ab} | 23.7 ± 1.3^{ab} | 22.9 ± 1.2^{b} |

| | | | | |
|---|---|---|---|---|
| There are significant differences (p < 0.05) between the numerical values with different alphabet superscripts. The numerical values represent mean ± standard deviation. | | | | |

According to the results, liver total cholesterol levels were significantly low in barley groups compared to polished rice group and wheat group (Table 4). Triglyceride levels showed a similar tendency to be significantly low in barley groups.

Table 5 shows organ weights of the rats in each group.

**[Table 5]**

| | Polished rice | Wheat | Barley 1 | Barley 2 |
|---|---|---|---|---|
| Liver (g) | 5.55 ± 0.33 | 5.85 ± 0.24 | 5.71 ± 0.29 | 5.51 ± 0.38 |
| Pancreas (g) | 0.54 ± 0.04 | 0.54 ± 0.04 | 0.58 ± 0.07 | 0.54 ± 0.05 |
| Small intestine (g) | 2.50 ± 0.32^{a} | 2.80 ± 0.30^{ab} | 3.20 ± 0.44^{b} | 3.15 ± 0.67^{b} |
| Total cecum weight (g) | 1.71 ± 0.21 | 2.04 ± 0.30 | 2.83 ± 1.09 | 2.47 ± 1.19 |
| Cecal wall (g) | 0.46 ± 0.09^{a} | 0.52 ± 0.07^{ab} | 0.64 ± 0.07^{b} | 0.61 ± 0.16^{b} |
| Large intestine (g) | 0.54 ± 0.03 | 0.61 ± 0.04 | 0.60 ± 0.04 | 0.61 ± 0.09 |
| Posterior abdominal | 0.94 ± 0.13^{a} | 0-85 ± 0.14^{ab} | 0.74 ± 0.17^{ab} | 0.68 ± 0.25^{b} |
| wall fat (g) | | | | |
| Periepididymal fat (g) | 1.46 ± 0.15 | 1.39 ± 0.12 | 1.24 ± 0.14 | 1.22 ± 0.28 |

| | | | | |
|---|---|---|---|---|
| There are significant differences (p <0.05) between the numerical values with different alphabet superscripts. The numerical values represent mean ± standard deviation. | | | | |

The diet-fed rats were dissected, and the organ weights were measured. According to the results (Table 5), the weights of small intestine and cecal wall were significantly increased in barley groups compared to polished rice group, thus indicating enhanced digestive function. The weight of posterior abdominal wall fat (visceral fat) tended to be low in barley groups, and particularly in barley group 2, it was significantly low compared to polished rice group. These results demonstrate that barley inhibits elevation of non-HDL cholesterol while also having an inhibiting effect on visceral fat accumulation.

### (Example 2: Cholesterol-improving effect of barley in hyperlipidemic mice)

Next, STR mice with naturally occurring hyperlipidemia (4 weeks old, male; Charles River Laboratories Japan, Inc.) were used to examine the effects of barley flour on total serum cholesterol levels. After one week of preliminary feeding, the STR mice were divided into 3 groups of 6 each, and the following test diet was fed for 2 months.

The compositions of the diets used for the feeding were based on the composition (modified AIN-93G) shown in Table 1. As for the lipid source, 10% lard was contained instead of 7% soybean oil (see "Project Report on Rat Response to High-Cholesterol Diet", Journal of the Japanese Society of Nutrition and Food Science, 45, 6, 564-567, 1992).

Wheat (unpolished) and two types of barley (unpolished) to be added to the diets were boiled. They were then freeze-dried, and ground with a cyclone mill into wheat flour and barley flour, respectively, for addition to the diets. With regard to wheat group and barley group, the diet was formulated to contain 5% total dietary fiber, the protein, lipid and total dietary fiber contents were adjusted, and the total weight was adjusted to be 1000 g using α-cornstarch.

The results of the formulation were as follows: The (1→3),(1→4)-β-D-glucan contents in the samples were 0.24% for wheat group, 2.23% for barley group 1 and 2.91% for barley group 2. That is, the proportions of (1→3),(1→4)-β-D-glucan in the dietary fiber of the samples were: wheat group = 4.8%, barley group 1 = 44.6%, barley group 2 = 58.2%.

The rats were reared in an environment with a temperature of 23 ± 2°C, humidity of 55 ± 5% and light/dark cycle of 12 hours, and the feed and water were made freely available.

The results are shown in Tables 6, 7, 8, 9 and 10. Table 6 shows initial body weight (g), final body weight (g), body weight gain (g/d), feed intake (g/d) and feed efficiency (%) for the STR mice in each group. The initial body weight (g) is the weight of each STR mouse at the start of feeding, the final body weight (g) is the weight of each STR mouse at the end of 2 weeks of feeding, the body weight gain (g/d) is the body weight increase per day of each STR mouse during the 2-week feeding period, the feed intake (g/d) is the dietary intake per day of each STR mouse during the 2-week feeding period, and the feed efficiency (%) is the ratio of feed intake to body weight gain.

**[Table 6]**

| | Wheat | Barley 1 | Barley 2 |
|---|---|---|---|
| Initial body weight (g) | 21.9 ± 1.6 | 21.9 ± 1.6 | 21.9 ± 0.8 |
| Final body weight (g) | 40.0 ± 1.8 | 38.6 ± 2.4 | 40.0 ± 1.1 |
| Body weight gain (g/d) | 0.31 ± 0.04 | 0.29 ± 0.04 | 0.31 ± 0.02 |
| Feed intake (g/d) | 3.90 ± 0.25 | 3.71 ± 0.38 | 3.97 ± 0.24 |
| Feed efficiency (%) | 8.06 ± 0.25 | 7.75 ± 0.93 | 7.89 ± 0.58 |

The numerical values in the table represent mean ± standard deviation.

According to the results, no difference was found between the groups in terms of body weight gain and other items during the 2-week feeding period (Table 6).

Table 7 shows the values of serochemical parameters of the STR mice in each group.

**[Table 7]**

| | Wheat | Barley 1 | Barley 2 |
|---|---|---|---|
| Total Cholesterol (mg/dl) | 216.0 ± 23.5 | 198.5 ± 31.8 | 183.4 ± 25.9 |
| HDL cholesterol, (mg/dl) | 165.6 ± 27.2 | 168.5 ± 17.8 | 188.7 ± 26.3 |
| Non-HDL cholesterol (mg/dl) | 50.5 ± 10.6^{a} | 31.3 ± 20.8^{b} | 7.2 ± 11,4^{c} |
| Triglycerides (mg/dl) | 174.7 ± 23.0 | 164.9 ± 30.6 | 140.2 ± 17.4 |
| Free fatty acids (mEq/l) | 0.779 ± 0.085 | 0.667 ± 0.067 | 0.655 ± 0.116 |
| Leptin (ng/ml) | 79.4 ± 36.0 | 64.3 ± 36.2 | 35.2 ± 8.3 |

In the table, the numerical values represent mean ± standard deviation. There are significant differences (p < 0.05) between the numerical values with alphabet superscripts. No statistically significant difference is found between the numerical values with no alphabet superscripts.

According to the results, total serum cholesterol levels were elevated in wheat group, but tended to be suppressed in barley groups. On the other hand, HDL cholesterol levels tended to be higher in barley groups, while non-HDL cholesterol levels were significantly low in barley groups 1 and 2 compared to wheat group. The levels of triglycerides and free fatty acids also tended to be lower in barley groups, thus demonstrating an overall improving effect on serum lipids. Serum leptin levels were also low, though not significantly, in barley group 2, and body fat was presumed to have been reduced (Table 7).

Table 8 shows organ weights of the STR mice in each group.

**[Table 8]**

| | Wheat | Barley 1 | Barley 2 |
|---|---|---|---|
| Live weight (g) | 40.0 ± 1.8 | 38.6 ± 2.4 | 40.0 ± 1.1 |
| Liver (g) | 2.14 ± 0.29 | 2.06 ± 0.29 | 2.12 ± 0.12 |
| Posterior abdominal | 0.75 ± 0.15^{a} | 0.64 ± 0.09^{ab} | 0.53 ± 0.15^{b} |
| wall fat (g) | | | |
| Periepididymal fat (g) | 0.88 ± 0.16 | 0.85 ± 0.10 | 0.86 ± 0.11 |

In the table, the numerical values represent mean ± standard deviation. There are significant differences (p < 0.05) between the numerical values with alphabet superscripts. No statistically significant difference is found between the numerical values with no alphabet superscripts.

According to the results of measurement of the organ weights (Table 8), the weight of posterior abdominal wall fat (visceral fat) tended to be low in barley groups, and particularly in barley group 2, it was significantly low compared to wheat group. These results demonstrate that barley inhibits elevation of non-HDL cholesterol while also having an inhibiting effect on visceral fat accumulation.

Table 9 shows the size and number of adipocytes for the STR mice in each group.

**[Table 9]**

| | Wheat | Barley 1 | Barley 2 |
|---|---|---|---|
| Posterior abdominal wall | 96.3 ± 4.8^{a} | 88.2 ± 6.9^{ab} | 87.7 ± 5.2^{b} |
| adipocyte size (µm) | | | |
| Posterior abdominal wall | 470 ± 67^{a} | 364 ± 85^{ab} | 356 ± 63^{b} |
| adipocyte volume (pL) | | | |
| Posterior abdominal wall | 1.68 ± 0.29 | 1.94 ± 0.46 | 1.62 ± 0.67 |
| adipocyte count (×10⁶) | | | |
| Periepididymal | 91.1 ± 5.0 | 90.8 ± 5.5 | 92.8 ± 6.4 |
| adipocyte size (µm) | | | |
| Periepididymal | 399 ± 68 | 396 ± 71 | 423 ± 78 |
| adipocyte volume (pL) | | | |
| Periepididymal | 2.38 ± 0.68 | 2.35 ± 0.63 | 2.22 ± 0.54 |
| adipocyte count (×10⁶) | | | |

In the table, the numerical values represent mean ± standard deviation. There are significant differences (p < 0.05) between the numerical values with alphabet superscripts. No statistically significant difference is found between the numerical values with no alphabet superscripts.

According to the results of examination of the inhibiting effect on adipocyte hypertrophy (Table 9), the size of posterior abdominal wall adipocytes is significantly small in barley groups, especially barley group 2, compared to wheat group. On the other hand, no significant difference was found between the groups in terms of adipocyte count. Thus, the reduction in weight of posterior abdominal wall fat seen in barley group 2 is presumed to be due to inhibition of adipocyte hypertrophy, and an improving effect on metabolic syndrome associated with adipocyte hypertrophy is expected.

Table 10 shows the amounts of fecal bile acids in the STR mice in each group.

**[Table 10]**

| | Wheat | Barley 1 | Barley 2 |
|---|---|---|---|
| Total bile acids (µmol/day) | 1.27 ± 0.13^{a} | 2.04 ± 0.55^{b} | 1.95 ± 0.43^{b} |

In the table, the numerical values represent mean ± standard deviation. There are significant differences (p < 0.05) between the numerical values with alphabet superscripts. No statistically significant difference is found between the numerical values with no alphabet superscripts.

According to the results, bile acid excretion was significantly increased in barley groups compared to wheat group (Table 10). This suggests that the non-HDL cholesterol-lowering effect of barley exhibited in the absence of cholesterol was exerted through bile acid metabolism.

### (Example 3: Effect of barley on intraperitoneal fat accumulation in KK mice)

The effect of barley flour on fat accumulation seen in Examples 1 and 2 was examined using KK mice which will be rendered insulin resistant by overfeeding and have intraperitoneal fat accumulation. After one week of preliminary feeding to the experimental animals (4-week-old males), they were divided into 4 groups of 8 each, and the test diet was fed for 2 months. The diets and conditions used for the feeding were the same as in Example 2, but a group fed only the basic diet containing neither wheat nor barley was used as the control group.

Table 11 shows initial body weight (g), final body weight (g), body weight gain (g/d), feed intake (g/d) and feed efficiency (%) for the KK mice in each group. The initial body weight (g) is the weight of each KK mouse at the start of feeding, the final body weight (g) is the weight of each KK mouse at the end of 2 weeks of feeding, the body weight gain (g/d) is the body weight increase per day of each KK mouse during the 2-week feeding period, the feed intake (g/d) is the feed intake per day of each KK mouse during the 2-week feeding period, and the feed efficiency (%) is the ratio of feed intake to body weight gain.

**[Table 11]**

| | Control | Wheat | Barley 1 | Barley 2 |
|---|---|---|---|---|
| Initial body weight (g) | 22.6 ± 0.8 | 22.6 ± 1.0 | 22.6 ± 0.9 | 22.7 ± 0.9 |
| Final body weight (g) | 37.9 ± 3.0 | 39.4 ± 0.9 | 39.7 ± 1.6 | 38.6 ± 0.9 |
| Body weight gain (g/d) | 0.27 ± 0.04 | 0.29 ± 0.02 | 0.29 ± 0.03 | 0.27 ± 0.02 |
| Feed intake (g/d) | 4.29 ± 0.46 | 4.20 ± 0.30 | 4.15 ± 0.39 | 4.39 ± 0.21 |
| Feed efficiency (%) | 6.17 ± 0.85^{a} | 6.92 ± 0.45^{ab} | 7.12 ± 0.73^{b} | 6.25 ± 0.54^{ab} |

In the table, the numerical values represent mean ± standard deviation. There are significant differences (p < 0.05) between the numerical values with alphabet superscripts. No statistically significant difference is found between the numerical values with no alphabet superscripts.

According to the results, no difference was found between the groups in terms of feed intake and body weight gain, and growth of the mice in all of the groups was excellent. However, feed efficiency is significantly high in barley group 1 compared to the control group (Table 11).

Table 12 shows the values of serochemical parameters of the KK mice in each group.

**[Table 12]**

| | Control | Wheat | Barley 1 | Barley 2 |
|---|---|---|---|---|
| Total cholesterol (mg/dl) | 81.6 ± 10.2 | 87.1 ± 16.0 | 95.2 ± 18.6 | 83.0 ± 9.7 |
| HDL cholesterol, (mg/dl) | 90.6 ± 13.7 | 74.1 ± 19.4 | 91.0 ± 16.9 | 81.7 ± 10.0 |
| Triglycerides (mg/dl) | 188.9 ± 75.5 | 193.3 ± 69.5 | 189.8 ± 46.7 | 178.1 ± 57.7 |
| Free fatty acids (mEq/l) | 0.733 ± 0.103^{a} | 0.688 ± 0.125^{a} | 0.700 ± 0.093^{a} | 0.486 ± 0.069^{b} |
| Blood sugar (mg/dl) | 319 ± 51 | 301 ± 84 | 316 ± 69 | 290 ± 86 |
| Leptin (ng/ml) | 121 ± 55 | 122 ± 61 | 106 ± 44 | 83 ± 28 |

In the table, the numerical values represent mean ± standard deviation. There are significant differences (p < 0.05) between the numerical values with alphabet superscripts. No statistically significant difference is found between the numerical values with no alphabet superscripts.

According to the results, total serum cholesterol levels in all of the groups were within the normal range and were not elevated (Table 12). There was also no effect observed in barley groups. The levels of triglycerides and free fatty acids tended to be lower in barley groups, while free fatty acid levels were significantly low in barley group 2 compared to the other groups. Serum leptin levels were also low, though not significantly, in barley groups, and this was presumed to reflect a reduction in body fat.

Table 13 shows hepatic lipid levels of the KK mice in each group.

**[Table 13]**

| | Control | Wheat | Barley 1 | Barley 2 |
|---|---|---|---|---|
| Total cholesterol (mg/ liver) | 6.89 ± 1.59^{a} | 3.84 ± 0.59^{b} | 3.81 ± 0.63^{b} | 3.23 ± 1.08^{b} |
| T triglycerides (mg/ liver) | 46.6 ± 19.0 | 31.2 ± 9.6 | 41.8 ± 12.1 | 35.8 ± 10.7 |

In the table, the numerical values represent mean ± standard deviation. There are significant differences (p < 0.05) between the numerical values with alphabet superscripts. No statistically significant difference is found between the numerical values with no alphabet superscripts.

According to the results, liver cholesterol levels were significantly low in wheat group and barley groups compared to the control group. No significant difference was found between the groups in terms of liver triglycerides (Table 13).

Table 14 shows organ weights of the KK mice in each group.

**[Table 14]**

| | Control | Wheat | Barley 1 | Barley 2 |
|---|---|---|---|---|
| Live weight (g) | 38.8 ± 1.0 | 39.4 ± 0.9 | 39.7 ± 1.6 | 38.6 ± 0.9 |
| Liver (g) | 1.70 ± 0.19 | 1.5 ± 0.11 | 1.69 ± 0.10 | 1.64 ± 0.14 |
| Posterior abdominal wall fat (g) | 0.60 ± 0.07^{ab} | 0.66 ± 0.08^{a} | 0.52 ± 0.10^{bc} | 0.45 ± 0.08^{c} |
| Periepididymal fat (g) | 1.29 ± 0.10 | 1.33 ± 0.17 | 1.32 ± 0.14 | 1.18 ± 0.17 |

In the table, the numerical values represent meant standard deviation. There are significant differences (p < 0.05) between the numerical values with alphabet superscripts. No statistically significant difference is found between the numerical values with no alphabet superscripts.

According to the results of measurement of the organ weights (Table 14), accumulation of posterior abdominal wall fat was significantly suppressed in barley groups compared to wheat group. A significant difference was also found between barley group 2 and the control group.

Table 15 shows the size and number of adipocytes for the KK mice in each group.

**[Table 15]**

| | Control | Wheat | Barley 1 | Barley 2 |
|---|---|---|---|---|
| Posterior abdominal wall adipocytes size (µm) | 96.9 ± 8.1^{a} | 90.0 ± 8.8^{ab} | 86.4 ± 9.1^{ab} | 85.4 ± 3.2^{b} |
| Posterior abdominal wall adipocyte volume (pL) | 485 ± 120^{a} | 391 ± 107^{ab} | 347 ± 100^{b} | 327 ± 37^{b} |
| Posterior abdominal wall adipocyte count (×10⁶) | 1.39 ± 0.43 | 1.96 ± 0.88 | 1.71 ± 0.61 | 1.47 ± 0.31 |
| Periepididymal adipocytes size (µm) | 98.2 ± 4.1 | 98.2 ± 4.0 | 102.9 ± 3.7 | 100.0 ± 3.7 |
| Periepididymal adipocyte volume (pL) | 498 ± 61 | 498 ± 60 | 573 ± 66 | 525 ± 59 |
| Periepididymal adipocyte count (×10⁶) | 2,77 ± 0.44 | 2.88 ± 0.65 | 2.45 ± 0.37 | 2.42 ± 0.52 |

In the table, the numerical values represent mean ± standard deviation. There are significant differences (p < 0.05) between the numerical values with alphabet superscripts. No statistically significant difference is found between the numerical values with no alphabet superscripts.

According to the results of examination of the inhibiting effect on adipocyte hypertrophy (Table 15), the size of posterior abdominal wall adipocytes is significantly small in barley groups, especially barley group 2, compared to the control group. No difference was found between wheat group and the control group. No difference was found between the groups in terms of adipocyte count. Thus, the reduction in weight of posterior abdominal wall fat seen in barley groups is presumed to be due to inhibition of adipocyte hypertrophy, and an improving effect on metabolic syndrome associated with adipocyte hypertrophy is expected.

Table 16 shows the amounts of total fecal lipids and bile acids in the KK mice in each group.

**[Table 16]**

| | Contol | Wheat | Barley 1 | Barley 2 |
|---|---|---|---|---|
| Total lipids (mg/day) | 11 ± 1^{a} | 21 ± 2^{b} | 19 ± 3^{b} | 21 ± 5^{b} |
| Total bile acids (µmol/day) | 1.92 ± 0.33 ^{ab} | 1.62 ± 0.17^{a} | 2.26 ± 0.34^{b} | 1.98 ± 0.64^{ab} |
| Apparent lipid absorption (%) | 96.4 ± 0.4^{a} | 91.9 ± 1.6^{b} | 91.9 ± 1.9^{b} | 93.1 ± 1.5^{b} |

In the table, the numerical values represent mean ± standard deviation. There are significant differences (p < 0.05) between the numerical values with alphabet superscripts. No statistically significant difference is found between the numerical values with no alphabet superscripts.

According to the results, total fecal lipids were significantly increased in wheat group and barley groups compared to the control group, and apparent lipid absorption was significantly low. Total fecal bile acid excretion was increased in barley groups compared to wheat group, but no significant difference was found compared to the control group (Table 16).

### (Example 4: Effect of barley on clinical parameters affecting metabolic syndrome symptoms in humans)

The following clinical trial was conducted with 39 adult males of age 30 to 60 (placebo group: 20, test group: 19) which had relatively high total cholesterol, LDL cholesterol and Body Mass Index (hereinafter referred to as BMI) and were not routinely taking any drugs or health foods that might affect lipid metabolism. BMI is a body weigh (physique) index calculated as weight (kg) ÷ height (m) ÷ height (m). A higher BMI is judged to mean that the degree of obesity is higher.

The clinical trial design was a double-blind, parallel-group, comparative study, wherein a designated assigner who was not directly involved in the trial randomly assigned subjects to test group and placebo group so that there would be no large differences between the two groups in terms of total cholesterol, LDL cholesterol, BMI and age, and wherein the assignment list was kept under lock and key until opening.

During the trial period, the subjects in placebo group (control group) were fed retort packed rice, and the subjects in test group were fed retort packed barley rice containing 50% barley. The test foods are fed in an amount of 2 packs per day for a period of 12 weeks. However, if the amount of test food was insufficient compared to the amount of staple food consumed before start of the trial, supplementation with polished rice, noodles or bread was permitted. Subsidiary foods and snacks were also allowed without any particular restrictions so that the eating habits and calorie intake were not greatly different than they were before start of the trial.

Table 17 shows calories for each pack of retort packed rice and retort packed barley rice, and analysis values for the components.

**[Table 17]**

| | Retort packed rice (placebo group) | Retort packed barley rice (test group) |
|---|---|---|
| Calories (Kcal) | 211 | 216 |
| Water (g) | 86.6 | 104.2 |
| Protein (g) | 3.3 | 5.0 |
| Lipids (g) | 0.6 | 0.5 |
| Carbohydrates (g) | 48.4 | 45.8 |
| Dietary fiber (g) | less than 0.7 | 4.5 |
| Water-soluble β-glucan (g) | 0.6 | 3.51 |
| Sodium (mg) | 1 | 3 |

The retort packed rice and retort packed barley rice were designed to have approximately the same calorie content, and the water-soluble β-glucan contents per pack were 0.6 g for the retort packed rice and 3.51 g for the retort packed barley rice.

The examination items were total serum cholesterol, LDL cholesterol, body weight, BMI, waist circumference, visceral fat, subcutaneous fat and total body fat (visceral fat + subcutaneous fat), and the examinations were performed before start of the trial and at 4, 8 and 12 weeks after start of the trial.

### 1) Cholesterol and LDL cholesterol

Table 18 shows the mean and standard deviation for total serum cholesterol and LDL cholesterol in placebo group and test group.

**[Table 18]**

| Item | Group | Before trial start | 4 weeks after trial start | 8 weeks after trial start | 12 weeks after trial start |
|---|---|---|---|---|---|
| Total cholesterol | Placebo | 246 ± 26.9 | 242 ± 26.2 | 242 ± 23.7 | 243 ± 27.9 |
| (mg/dL) | Test | 235 ± 21.7 | 231 ± 26.5 | 218 ± 29.4 | 224 ± 33.9 |
| LDL-cholesterol | Placebo | 160.0 ± 23.9 | 156.9 ± 19.9 | 163.4 ± 25.1 | 159.5 ± 24.4 |
| (mg/dL) | Test | 153.4 ± 16.4 | 143.9 ± 18.5 | 141.9 ± 22.7 | 147.7 ± 27.7 |

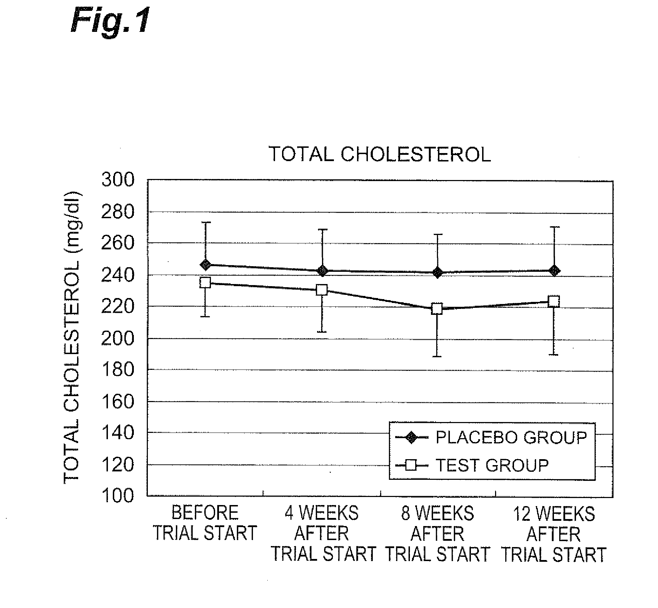

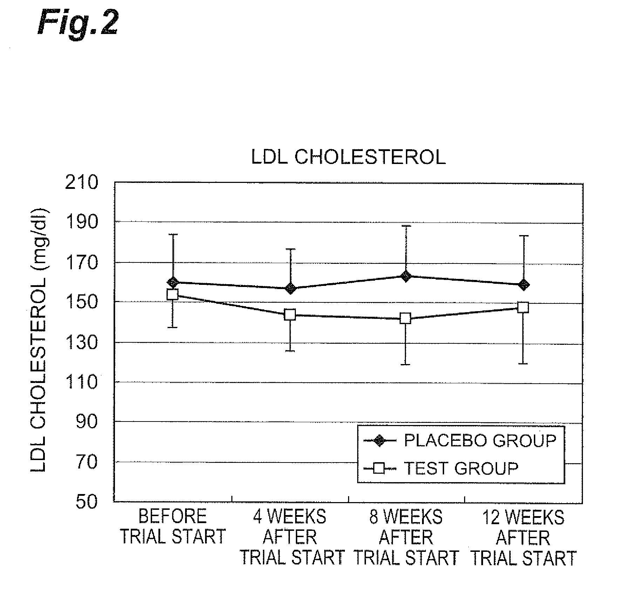

Fig. 1 is a graph showing changes in total serum cholesterol with time in placebo group and test group. Fig. 2 is a graph showing changes in total serum LDL cholesterol with time in placebo group and test group.

Repeated measures analysis of variance was performed on each day of examination, and the error variance was estimated. As a result, total serum cholesterol and LDL cholesterol tended to be low in test group compared to placebo group, and the F-test results showed that there was a statistically significant difference (p < 0.05) between the two groups in terms of either item.

### 2) Body weight, BMI, waist circumference, visceral fat and total body fat

Table 19 shows the mean and standard deviation for body weight, BMI, waist circumference, visceral fat, subcutaneous fat and total body fat (visceral fat + subcutaneous fat) in placebo group and test group.

**[Table 19]**

| Item | Group | Before trial start | 4 weeks after trial start | 8 weeks after trial start | 12 weeks after trial start |
|---|---|---|---|---|---|
| Body weight | Placebo | 71.4 ± 6.7 | 71.3 ± 6.7 | 71.2 ± 6.6 | 71.2 ± 6.7 |
| (kg) | Test | 75.1 ± 10.4 | 74.7 ± 10.7 | 74.4 ± 10.6 | 74.1 ± 10.4 |
| BMI | Placebo | 24.5 ± 2.4 | 24.4 ± 2.3 | 24.5 ± 2.3 | 24.5 ± 2.4 |
| | Test | 26.2 ± 2.8 | 26.0 ± 2.9 | 26.0 ± 2.9 | 25.9 ± 2.9 |
| Body fat percentage | Placebo | 24.1 ± 4.5 | 24.1 ± 4.5 | 24.8 ± 4.7 | 25.5 ± 4.8 |
| (%) | Test | 25.5 ± 5.9 | 25.5 ± 5.4 | 26.1 ± 5.9 | 25.9 ± 5.7 |
| Waist circumference | Placebo | 87 ± 6.8 | 87 ± 6.8 | 87 ± 6.5 | 87 ± 6.5 |
| (cm) | Test | 91 ± 7.7 | 90+7.6 | 90 ± 7.5 | 90 ± 8.1 |
| Visceral fat | Placebo | 77 ± 28.7 | 81 ± 38.0 | 76 ± 35.1 | 76 ± 33.6 |
| (cm²) | Test | 102 ± 41.2 | 97 ± 37.9 | 95 ± 36.2 | 92 ± 36.8 |
| Subcutaneous fat | Placebo | 157 ± 58.7 | 161 ± 61.0 | 159 ± 61.6 | 157 ± 60.2 |
| (cm²) | Test | 171 ± 72.2 | 168 ± 69.1 | 170 ± 72.9 | 166 ± 70.6 |
| Total body fat | Placebo | 235 ± 69.5 | 241 ± 78.1 | 236 ± 78.3 | 233 ± 73.9 |
| (cm²) | Test | 273 ± 94.2 | 266 ± 90.3 | 265 ± 95.3 | 258 ± 90.3 |

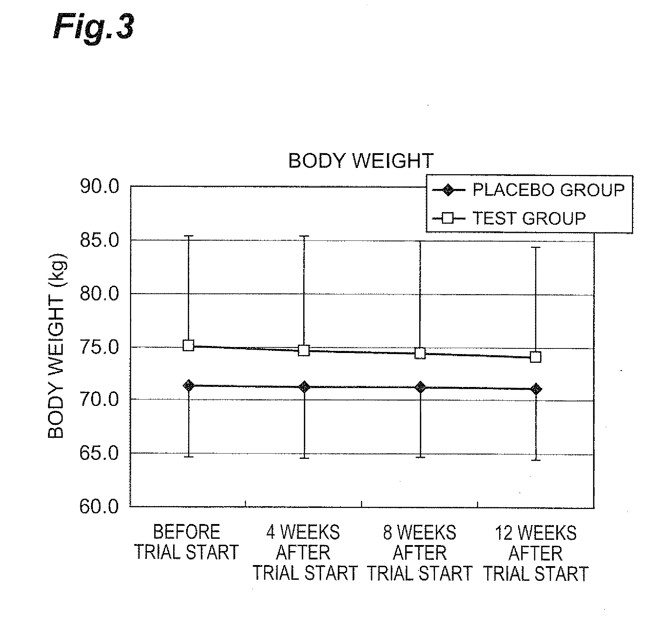

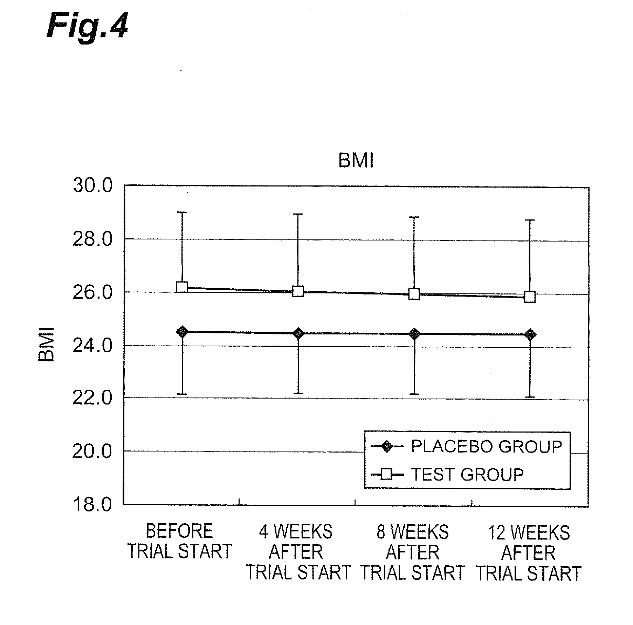

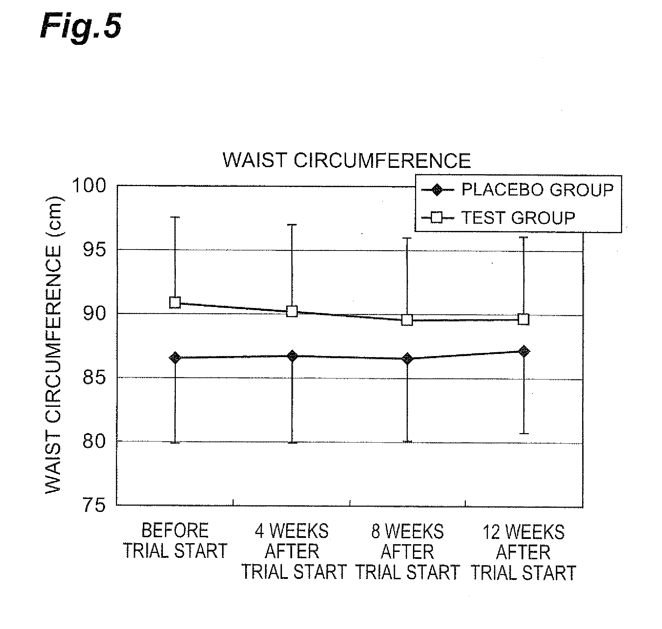

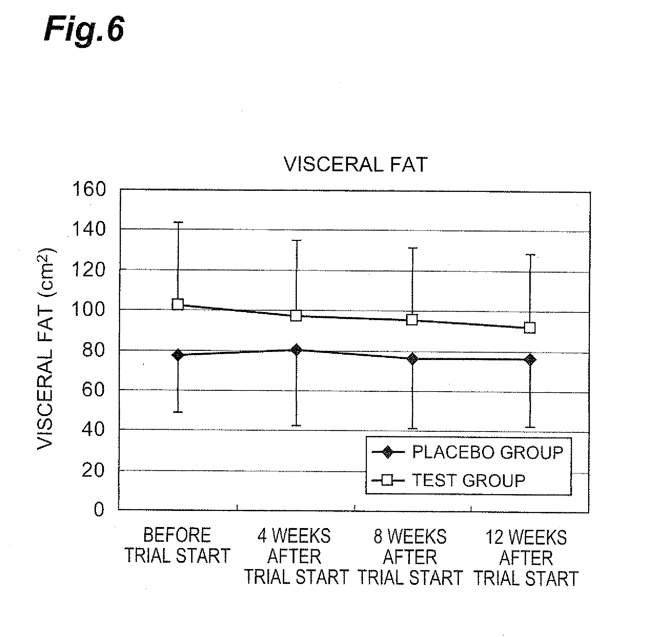

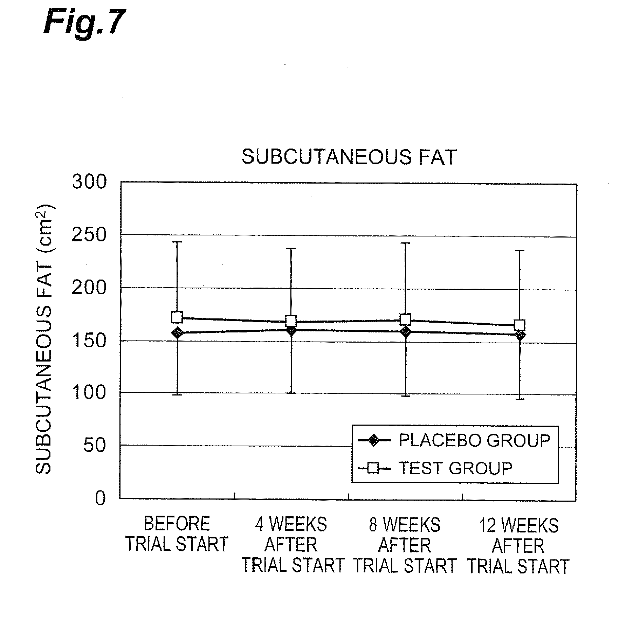

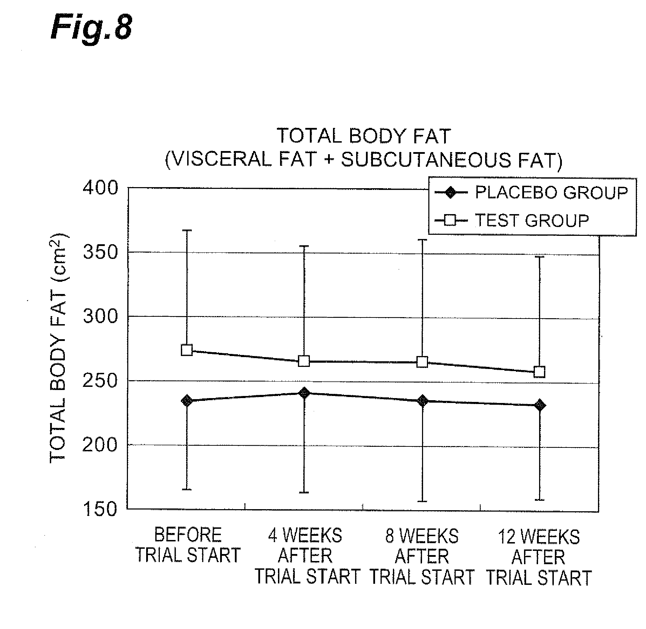

Figs. 3 to 8 are graphs showing changes in body weight, BMI, waist circumference, visceral fat, subcutaneous fat and total body fat (visceral fat + subcutaneous fat) with time in placebo group and test group.

For each examination item, the measured values at the end of the feeding (12th week), which was the endpoint of the trial, were compared between the groups by unpaired t-test, using residual variance independently of F-test. Since a high correlation was found between the values before feeding and the values at the endpoint, statistical analysis was performed with the values before feeding added to the model as covariates.

According to the results, a statistically significant reduction (p<0.05) was found in test group compared to placebo group for all of the items including body weight, BMI, waist circumference, body fat percentage, visceral fat and total body fat (visceral fat + subcutaneous fat). Since waist circumference and visceral fat are diagnostic criteria for metabolic syndrome, these results demonstrate that intake of barley has a curative effect on metabolic syndrome in humans.

No major difference was found between the retort packed rice and retort packed barley rice in terms of calories and water, protein, lipid, carbohydrate and sodium contents of each pack, while the retort packed barley rice contained notably large amounts of dietary fiber and water-soluble β-glucan compared to the retort packed rice. Therefore, the results described above strongly suggest that the dietary fiber and water-soluble β-glucan in barley have a curative effect on metabolic syndrome in humans.

### Industrial Applicability

The therapeutic agent for metabolic syndrome of the invention and foods containing it can reduce visceral fat accumulation to prevent or treat metabolic syndrome.

## Claims

1. A therapeutic agent for metabolic syndrome comprising, as an active ingredient, dietary fiber aggregation in which water-soluble β-glucan has the highest content.

2. The therapeutic agent for metabolic syndrome according to claim 1, wherein the water-soluble β-glucan is water-soluble β-glucan having a β-1,3 glycoside bond and β-1,4 glycoside bond in the same molecule.

3. The therapeutic agent for metabolic syndrome according to claim 1 or 2, wherein the dietary fiber aggregation is obtained from one or more sources selected from the group consisting of barley, oats, rye and adlay.

4. The therapeutic agent for metabolic syndrome according to any one of claims 1 to 3, wherein the water-soluble β-glucan content is 20% to 100% by weight relative to the total weight of the dietary fiber aggregation.

5. The therapeutic agent for metabolic syndrome according to any one of claims to 4, which has the effect of reducing visceral fat and/or the effect of inhibiting visceral fat accumulation.

6. The therapeutic agent for metabolic syndrome according to any one of claims 1 to 5, which has the effect of inhibiting adipocyte hypertrophy.

7. A food containing the therapeutic agent for metabolic syndrome according to any one of claims 1 to 6.
